# EUROPÄISCHE PATENTSCHRIFT

(11) **EP 1 524 260 B1**
(45) Veröffentlichungstag und Bekanntmachung des Hinweises auf die Patenterteilung: **28.03.2007**
(21) Anmeldenummer: 04023291.0
(22) Anmeldetag: 30.09.2004
(51) Int. Cl.: C07C 209/78, C07C 263/10, C07C 265/14

(54) **Verfahren zur Herstellung von Di- und Polyaminen der Diphenylmethanreihe**
Process for the preparation of di- and polyamines of the diphenyl methane series
Procédé de préparation de di- et polyamines de la série du diphénylméthane

(30) Priorität: 13.10.2003 DE 10347438
(43) Veröffentlichungstag der Anmeldung: 20.04.2005
(73) Patentinhaber: Bayer MaterialScience AG, 51368 Leverkusen (DE)
(72) Erfinder: Dugal, Markus Dr, 47906 Kempen (DE)

(56) Entgegenhaltungen:
- EP-A- 0 083 791
- EP-A- 1 167 343
- EP-A- 1 344 766
- US-B1- 6 262 307

## Beschreibung

Die vorliegende Erfindung betrifft ein Verfahren zur Herstellung von Di- und Polyaminen der Diphenylmethanreihe (MDA), enthaltend den Schritt der simultanen Herstellung von Anilin und Formaldehyd aus den Ausgangsverbindungen Nitrobenzol und Methanol, bei dem durch eine katalytische Transferreduktion Nitrobenzol zu Anilin reduziert und gleichzeitig Methanol zu Formaldehyd oxidiert wird.

Anilin und Formaldehyd sind wichtige Zwischenprodukte u.a. für die Polymerindustrie. Anilin und Formaldehyd werden zum Beispiel gemeinsam als Ausgangsstoffe zur Herstellung von Methylendiphenyldiamin und den entsprechenden Polyaminen (MDA) und Methylendiphenyldiisocyanat und den entsprechenden Polyisocyanaten (MDI), einem wichtigen Monomer für die Polyurethanherstellung, eingesetzt. Für die Herstellung von Anilin und Formalin existieren jeweils eine Reihe von Verfahren, die zum Teil technisch ungesetzt wurden. Die industrielle Herstellung von Anilin erfolgt derzeit durch die katalytische Gasphasenhydrierung von Nitrobenzol mit Wasserstoff in adiabater (Hydrocarbon Process 59 (Nov. 1979) no. 11, 136; US-A-3,636,152) oder isothermer Fahrweise (US-A-4,265,834) unter Verwendung von Cu- oder Pd- Katalysatoren. Von untergeordneter Bedeutung sind die Reduktion von Nitrobenzol mit Eisen (Bechamp-Verfahren, *Winnacker-Küchler Chemische Technologie,* 3rd ed., Vol. **4**, pp. 170 - 171) und die heterogen katalysierte Gasphasen-Ammonolyse von Phenol (Halcon-Verfahren, US-A-3,272,865).

Die Herstellung von Formaldehyd im technischen Maßstab erfolgt derzeit im wesentlichen durch Silber-katalysierte Dehydrierverfahren (DE-A-2 322 757, US-A-2,519,788) und das sogenannte Formox-Vefahren (GB-A-1 080 508). EP 1 344 766 offenbart die Herstellung von Polyisocyanaten durch Umsetzung von Anilin und Formaldehyd in Gegenwart eines sauren Katalysators.

Bei den Silber-katalysierten Verfahren wird Methanol durch Luft bei > 600°C an einem Silberkatalysator unter Bildung von Formaldehyd und Wasserstoff dehydriert, wobei der Wasserstoff im weiteren Reaktionsverlauf bzw. in nachgeschalteten Reaktionsstufen mit Luftsauerstoff zwecks Energieerzeugung zu Wasser umgesetzt wird. Das Formox-Verfahren beinhaltet eine zweistufige Oxidation von Methanol zu Formaldehyd und Wasser (Oxidations-Reduktionszyklus des Katalysators), die bei niedrigeren Temperaturen im Bereich 270-300°C in der Regel unter Einsatz von Molybdän-Eisenkatalysatoren abläuft.

Aus der Anwendung der erwähnten Verfahren folgt zwangsläufig, dass die Produkte Anilin und Formaldehyd in getrennten Anlagen unabhängig voneinander hergestellt und aufgearbeitet werden müssen. Für die Herstellung von Anilin, insbesondere durch die technisch maßgeblichen Hydrierverfahren, muss dabei zudem Wasserstoff als kostenintensives Reduktionsmittel eingesetzt werden.

Für die Herstellung von MDA durch säurekatalysierte Umsetzung von Anilin und Formaldehyd wäre es von Vorteil, Anilin und Formaldehyd simultan in einem Verfahren herzustellen, um weniger Anlagenteile zu benötigen und dadurch Investitionen und Betriebskosten reduzieren zu können. Weiterhin wäre es im Hinblick auf die Materialkosten und die Sicherheit des Verfahrens von Vorteil, bei der Nitrobenzol-Reduktion zu Anilin den Hydrierwasserstoff durch eine günstigere und besser handhabbare Wasserstoffquelle zu ersetzen, die zudem Wasserstoff unter Entstehung eines Wertstoffes abgibt.

Die vorliegende Erfindung betrifft ein Verfahren zur Herstellung von Di- und Polyaminen der Diphenylmethanreihe, bei dem man
a) Nitrobenzol und Methanol in Gegenwart eines Katalysators zu Anilin und Formaldehyd umsetzt, und anschließend
b) das in Schritt a) hergestellte Anilin und Formaldehyd in Gegenwart eines sauren Katalysators zu Di- und Polyaminen der Diphenylmethanreihe umsetzt.

In Schritt a) wird Nitrobenzol durch eine katalytische Transferreduktion zu Anilin reduziert und gleichzeitig Methanol zu Formaldehyd oxidiert. Die Herstellung von Anilin durch katalytische Transferreduktion von Nitrobenzol mit Methanol ist von Rossi et al. (Gaz. Chim. It., 122, 1992, 221-223) beschrieben. Dabei wird ein Cu-Katalysator bei Temperaturen von 180° verwendet. Als Reaktionsprodukt wird bei einem Umsatz von 58% lediglich Anilin angegeben. Rossi et al. diskutieren jedoch die theoretische Möglichkeit, dass Formaldehyd, Methylformiat, CO und CO₂ als Nebenprodukte der Reaktion entstehen könnten. Ein experimenteller Nachweis dafür wird aber nicht gegeben. Die Möglichkeit, dass durch die Transferreduktion von Nitrobenzol mit Methanol ein Reaktionsproduktgemisch enthaltend Anilin und Formaldehyd mit einer Zusammensetzung erhalten werden können, die einen direkten Einsatz in der Herstellung von MDA erlauben, wird in Gaz. Chim. It., 122, **1992**, 221-223 nicht in Betracht gezogen.

Geeignete Katalysatoren für die Transferreduktion von Nitrobenzol in Schritt a) sind zum Beispiel anorganische, im Reaktionsmedium nicht lösliche (heterogene) Katalysatoren oder lösliche (homogene) Metallkomplexe oder Salze, wobei diese Katalysatoren ein oder mehrere Metalle als katalytisch wirksame Komponenten in elementarer oder gebundener Form enthalten. Geeignete Metalle sind beispielsweise Pd, Pt, Rh, Ir, Ru, Fe, Co, Ni, Cu, Al, Mg, Zr, Zn, V, Cr, Mo, W, Pb, Lanthanoide. Bevorzugt werden Pd, Pt, Ir, Ru, Cu, oder Fe- haltige Katalysatoren eingesetzt.

Die Umsetzung von Nitrobenzol mit Methanol in Schritt a) erfolgt vorzugsweise in Gegenwart von Hilfsstoffen. Geeignete Hilfsstoffe sind beispielsweise basische anorganische oder organische Verbindungen die löslich oder unlöslich im Reaktionsmedium sind oder Lösungsmittel. Geeignete Basen sind z.B. Hydroxide wie NaOH, KOH oder NH₄OH, Carbonate wie Na₂CO₃ oder K₂CO₃, Hydrogencarbonate wie NaHCO₃, Amine wie Triethylamin oder Anilin oder unlösliche basische Festsstoffe wie z.B. Hydrotalcite, Al₂O₃, MgO. Unlösliche basische Feststoffe können gegebenenfalls gleichzeitig als Base und als Trägermaterial für den Katalysator dienen. Bevorzugte Basen sind NaOH, KOH oder Hydrotalcite.

Geeignete Lösungsmittel sind beispielsweise Wasser, Alkohole, organische Amine und/oder Nitroverbindungen. Bevorzugte Lösungsmittel sind die an der Reaktion beteiligten Komponenten Methanol, Nitrobenzol, Wasser und Anilin.

Die Reaktion kann generell in der Gas- und/oder in der Flüssigphase durchgeführt werden. Geeignete Reaktionstemperaturen liegen üblicherweise im Bereich von 20°C - 500°C, bevorzugt im Bereich von 50°C - 300°C. Der absolute Reaktionsdruck liegt üblicherweise im Bereich von 0,1 bar bis 300 bar, bevorzugt im Bereich von 1bar bis 100 bar. Die Konzentrationen und Konzentrationsverhältnisse der Ausgangsverbindungen sowie der Hilfsstoffe können im Prinzip frei gewählt werden. Je nach Wahl der Reaktionsbedingungen kann in der Reaktion Teil- oder Vollumsatz bezogen auf Methanol oder Nitrobenzol erreicht werden. Neben den Zielprodukten Anilin und Formaldehyd sind unter anderen Ameisensäure, CO, CO₂, Carbonate, Ameisensäuremethylester, N-Formylanilin, N-Methylanilin sowie verschiedene Aminale und Halbaminale aus Anilin und Formaldehyd mögliche Nebenprodukte der Reaktion. Die Art und Konzentration der erzeugten Nebenkomponenten variiert dabei in Abhängigkeit vom eingesetzten Katalysator und den eingestellten Reaktionsbedingungen.

Die Reaktionsprodukte Anilin und Formaldehyd und die gegebenenfalls auftretenden Nebenkomponenten sowie nicht umgesetztes Nitrobenzol und Methanol sowie die eingesetzten Hilfsstoffe können teilweise oder vollständig aus der Reaktionsmischung abgetrennt und gegebenenfalls zu den Reinverbindungen aufgearbeitet werden. Auf diese Weise erhaltenes Anilin und / oder Formaldehyd kann neben der MDA-Herstellung prinzipiell auch anderen Anwendungen zugeführt werden. Gegebenenfalls isolierte Nebenverbindungen wie z.B. CO oder CO₂ stehen ebenfalls prinzipiell für andere Anwendungen zur Verfügung. Nicht umgesetztes Nitrobenzol und / oder Methanol werden vorzugsweise in den Reaktionskreislauf zurückgeführt.

Alternativ können die Reaktionsprodukte Anilin und Formaldehyd zusammen mit den nicht umgesetzten Ausgangsverbindungen Nitrobenzol und Methanol sowie gegebenenfalls den Hilfsstoffen vollständig oder teilweise in der Reaktionsmischung belassen werden und in Schritt b) direkt zu MDA umgesetzt werden.

In Schritt b) werden das in Schritt a) hergestellte Anilin und Formaldehyd gegebenenfalls nach vorheriger Aufreinigung weiter umgesetzt zu MDA. Dazu wird dem Gemisch enthaltend Anilin und Formaldehyd ein saurer Katalysator zugesetzt.

Geeignete saure Katalysatoren sind starke organische oder anorganische Säuren, beispielsweise Salzsäure, Schwefelsäure, Phosphorsäure, Methansulfonsäure oder feste Säuren wie z.B. Zeolithe. Bevorzugt wird Salzsäure eingesetzt.

Das Reaktionsgemisch wird im Regelfall nach einer Vermischungsphase und einer Vorreaktion im Temperaturbereich zwischen 20°C und 100°C, vorzugsweise im Temperaturbereich von 30°C bis 80°C in Stufen oder kontinuierlich und ggf. unter Überdruck auf eine Temperatur von 100°C bis 250°C bevorzugt auf 100°C bis 180°C, besonders bevorzugt auf eine Temperatur von 100°C bis 160°C gebracht.

Das danach erhaltene Reaktionsgemisch wird anschließend vorzugsweise mit einer Base neutralisiert und in einem Scheidebehälter die wässrige und die organische getrennt. Das MDA ist dann in der organischen Phase enthalten.

Die Erfindung betrifft weiterhin ein Verfahren zur Herstellung von Di - und Polyisocyanaten der Diphenylmethanreihe, bei dem man
a) Nitrobenzol und Methanol in Gegenwart eines Katalysators zu Anilin und Formaldehyd umsetzt, und anschließend
b) das in Schritt a) hergestellte Anilin und Formaldehyd in Gegenwart eines sauren Katalysators zu Di- und Polyaminen der Diphenylmethanreihe umsetzt, und anschließend
c) die in Schritt b) hergestellten Di- und Polyamine der Diphenylmethanreihe durch Phosgenierung zu den Di- und Polyisocyanaten der Diphenylmethanreihe umsetzt.

Dazu wird das in Schritt b) hergestellte MDA anschließend nach den bekannten Methoden mit Phosgen in einem inerten organischen Lösungsmittel zu den entsprechenden Isocyanaten umgesetzt. Das Molverhältnis von Roh-MDA aus Schritt b) zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird zweckmäßigerweise so bemessen, dass die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist. Nach beendeter Phosgenierung wird der Reaktionsmischung das überschüssige Phosgen, das inerte organische Lösungsmittel oder Mischungen davon durch Destillation abgetrennt.

### Beispiele

### 1) Beispiele für die simultane Herstellung von Anilin und Formaldehyd aus Nitrobenzol und Methanol durch Transferhydrierung

### Beispiele 1-20: Variation des Katalysators bei konstanten Reaktionsbedingungen - niedrige Temperatur

Die Versuchsergebnisse und relevante Ansatzdaten sind in Tabelle 1 zusammengefasst.

### Durchführung:

In einem Rundkolben wurden 2,402 g (20 mmol) Nitrobenzol vorgelegt und 0,281g (5 mmol) KOH gelöst in 15,8 g Methanol und 0,6 g Naphthalin (interner Standard) zugegeben. Zur Vorlage wurden unter Magnetrührung eine Katalysatormenge zugegeben, die 0,4 mmol Aktivmetall des jeweils eingesetzten Katalysators entspricht. Das Gemisch wurde in einem Ölbad (T = 86°C) unter Magnetrührung auf Rückflussbedingungen gebracht und nach einer bestimmten Reaktionsdauer eine Probe entnommen und diese filtriert. Ein Teil des Filtrats wurde gaschromatographisch bezüglich Anilin und Nitrobenzolgehalt analysiert (interner Standard Naphthalin). Ein anderer Teil des Filtrats wurde mit Hilfe des sogenannten Nash-Reagens (T. Nash, Biochem. J. 55, 416, **1953**) umgesetzt. Auf diese Weise konnte vorhandenes Formaldehyd photometrisch analysiert werden (Wellenlänge 408 nm).

### Beispiele 21-28: Variation unlöslicher basischer MgO-Trägermaterialien - hohe Temperatur

Die experimentellen Ergebnisse und relevanten Katalysatordaten sind in Tabelle 2 zusammengefasst.

### Durchführung

In ein Schraubgefäß aus V4A-Stahl mit Magnetrührer wurden 1,201 g (10 mmol) Nitrobenzol und 3,2 g Methanol zugegeben Zur Vorlage wurden unter Magnetrührung Katalysator entsprechend einer Menge von 0,2 mmol Pd geträgert auf MgO als unlöslicher Base (5 Gew.% Pd auf MgO) zugegeben. Das Gefäß wurde verschlossen und unter Magnetrührung in einem Ölbad auf 180°C aufgeheizt. Nach 3 h Reaktionszeit wurde nach Abkühlung der Reaktorinhalt filtriert. Ein Teil des Filtrats wurde mittels HPLC-chromatographie analysiert und ein anderer Teil mit Nash-Reagens umgesetzt und photometrisch analysiert (siehe oben)

**Tabelle 1*: Beispiele 1-20 Variation des Katalysators - niedrige Temperatur**

| **Nr.** | **Katalysator (% = Gewichtsprozent)** | **Metalle** | **Trägermaterial** | **t (h)** | **U [%]** | **S [%]** | **A [%]** | **A/F** |
|---|---|---|---|---|---|---|---|---|
| 1 | 10 % Pd/C | Pd | Aktivkohle (Aldrich) | 48 | 37,90 | 90,47 | 34,29 | 8,229460391 |
| 2 | 20g/L Cu auf Tonerde (Al₂O₃) | Cu | Tonerde (Condea) | 48 | 17,71 | 1,75 | 0,31 | 2,833531701 |
| 3 | 20g/L Ni auf Tonerde (Al₂O₃) | Ni | Tonerde (Condea) | 48 | 21,81 | 0,63 | 0,14 | 0,146611338 |
| 4 | 20g/L Ru auf Tonerde (Al₂O₃) | Ru | Tonerde (Condea) | 48 | 18,43 | 21,04 | 3,88 | 0,901105975 |
| 5 | 2% Pd auf Tonerde (Al₂O₃) | Pd | Tonerde (Condea) | 48 | 18,03 | 15,28 | 2,76 | 0,843336715 |
| 6 | 0,588 % Cu 0,216 % Fe 2,614 % Cr 0,560 % Mo 0,642% Zn 2 % NaOH auf Spheralite (=SPH) 501 (Al₂O₃) | Cu, Fe, Cr, Mo, Zn | Spheralite (=SPH) 501 (Al₂O₃, Rhodia) | 48 | 11,27 | 1,40 | 0,16 | 0,092437402 |
| 7 | 1,5 % Zn 1,5 % Cu auf Cr- Matrix | Cu, Zn | Cr-Oxid | 48 | 4,59 | 2,56 | 0,12 | 0,018546782 |
| 8 | 1,5 % Cu 1,5 % Pd auf Cr- Matrix | Pd, Cu | Cr-Oxid | 48 | 11,03 | 55,81 | 6,16 | 0,822392298 |
| 9 | 1,5% Cu 1,5% Zn 1,5% Pd auf Cr-Matrix | Pd, Cu, Zn | Cr-Oxid | 48 | 15,17 | 32,92 | 4,99 | 1,100314209 |
| 10 | 3%Pd + 5% NaOH auf SPH 501 (Al₂O₃) | Pd | SPH 501 (Rhodia) | 48 | 71,02 | 64,53 | 45,83 | 4,226413714 |
| 11 | 3%Ru + 5% NaOH auf SPH 501 (Al₂O₃) | Ru | SPH 501 (Rhodia) | 48 | 4,93 | 17,75 | 0,88 | 0,593633327 |
| 12 | 3% Rh + 5% NaOH auf SPH 501(Al₂O₃) | Rh | SPH 501 (Rhodia) | 48 | 20,31 | 22,78 | 4,63 | 1,492302699 |
| 13 | 3 %Pt + 5 %NaOH auf SPH 501 | Pt | SPH 501 (Rhodia) | 48 | 33,67 | 33,33 | 11,22 | 0,526037618 |
| 14 | 4 %Ru(Chlorid) auf MgO (30 mesh) Cl-frei gewaschen | Ru | MgO | 48 | 9,38 | 6,71 | 0,63 | 0,095063673 |
| 15 | 4 %Co(Chlorid) auf Spheralite 501 1,4-2,8mm Cl-frei | Co | SPH 501 (Rhodia) | 48 | 5,91 | 1,39 | 0,08 | 0,074885373 |
| 16 | 4 %Ni(Chlorid) auf Spheralite 501 (Al₂O₃, Rhodia) 1,4-2,8mm Cl-frei | Ni | SPH 501 (Rhodia) | 48 | 1,26 | 0,00 | 0,00 | 0 |
| 17 | 4 %Cu(Chlorid) auf Spheralite 501 (Al₂O₃, Rhodia) 1,4-2,8mm Cl-frei gewaschen | Cu | SPH 501 (Rhodia) | 48 | 3,28 | 27,46 | 0,90 | 1,184254739 |
| 18 | Hydro-Talcit Fe-MgAl (2,5% Fe) | Fe Mg Al | Hydrotalcit | 48 | 10,06 | 22,06 | 2,22 | 0,407727292 |
| 19 | Tris(triphenylphosphin)-ruthenium(II)-chlorid (10,5 % Ru) | Ru | Kein | 48 | 14,41 | 22,03 | 3,17 | 2,06875702 |
| 20 | 20g/L Ir auf Condea Tonerde (Al₂O₃) | Ir | Tonerde (Condea) | 24 | 30,69 | 56,45 | 17,32 | 1,915449027 |

| | | | | | | | | |
|---|---|---|---|---|---|---|---|---|
| U = Umsatz Nitrobenzol, S = Selektivität Anilin bezüglich Nitrobenzol, A= Ausbeute Anilin, A/F = molares Verhältnis Anilin: Formaldehyd, t = Reaktionsdauer | | | | | | | | |

**Tabelle 2: Beispiele 21-28: Variation des basischen Katalysatorträgers - hohe Temperatur**

| **Nr** | **Träger [MgO-Type]** | **Quelle** | **U [%]** | **S [%]** | **A [%]** | **A/F** |
|---|---|---|---|---|---|---|
| 21 | 325mesh /99,5% | Aldrich | 26,90 | 88,76 | 23,88 | 7,24 |
| 22 | 30mesh /98% | Aldrich | 1,22 | 93,98 | 1,15 | 1,01 |
| 23 | p.A./>97% | Acros Organics | 15,12 | 92,39 | 13,97 | 3,09 |
| 24 | A.R.G./>96% | Fisher Scientific | 39,959 | 94,177 | 37,633 | 6,75 |
| 25 | 100mesh /99,5% | ABCR | 33,888 | 96,266 | 32,623 | 17,72 |
| 26 | light/98-100,5% | Riedel de Haên | 18,329 | 90,342 | 16,558 | 4,17 |
| 27 | A.R.G. / >96% | Fisher Scientific | 17,39 | 92,04 | 16,00 | 2,65 |
| 28 | A.R.G. />96% | Fisher Scientific | 13,00 | 90,26 | 11,74 | 3,67 |

| | | | | | | |
|---|---|---|---|---|---|---|
| U = molarer Umsatz von Nitrobenzol, S = Selektivität Nitrobenzol zu Anilin , A= Ausbeute Anilin (bezogen auf Nitrobenzol), A/F = Anilin/Formalin-Verhältnis | | | | | | |

### 2) Beispiel für die Herstellung von MDA

Zur Herstellung der Anilin/Formalin-Reaktionsmischung wurden in einem Schraubgefäß aus V4A-Stahl, 2,402g (10 mmol) Nitrobenzol vorgelegt und 0,14 g (0,25 mmol) KOH gelöst in 8g (250 mmol) Methanol zugegeben. Zur Vorlage wurden unter Magnetrührung 0,2 g eines Pd-Aktivkohle-Katalysators (10 Gew.-% Pd, Firma Aldrich) zugegeben. Das Gefäß wurde verschlossen und in einem Ölbad mit einer Temperatur von 180°C platziert.

Die Reaktion wurde nach 3 h unterbrochen und das Gemisch vom Katalysator mittels Filtration abgetrennt und via HPLC (Tabelle 3) und Nash-Reaktion/Fotometrie (Ergebnis 0,15 Massen-% Formaldehyd) analysiert. Ein Teil (7 g) der Reaktionsmischung enthaltend 0,29 g (3,11 mmol) Anilin und 0,0106 g (0,35 mmol) Formaldehyd (molares Verhältnis Anilin zu Formaldehyd A/F = 8,9) wurde anschließend in einem Rundkolben mit Magnetrührer auf 45°C temperiert und eine für einen Protonierungsgrad von 18,5% (bezogen auf mol eingesetztes Anilin) notwendige Menge HCl (wässrige Lösung) zugegeben. Nach beendeter Zugabe wurde der Ansatz für 30 min bei 45°C gerührt, dann langsam auf Rückflusstemperatur (ca. 105°C) aufgeheizt und 10 h bei dieser Temperatur gerührt. Nach beendeter Reaktion wurde aus dem Umlagerungsgemisch eine Probe zur HPLC-Analytik entnommen. Die Analysenergebnisse sind in Tabelle 4 dargestellt.

**Tabelle 3: Via HPLC ermittelte Zusammensetzung des Reaktionsgemisches nach der Reaktion von Nitrobenzol mit Methanol (Angaben in Massenprozent)**

| Anilin [m%] | Nitrobenzol [m%] | N-Methylanilin [m%] |
|---|---|---|
| 4,19 | 8,91 | 0,08 |

**Tabelle 4: HPLC-Analyse der Reaktionsmischung nach MDA-Kondensation (Angaben in Massenprozent, nicht ausgewertet wurde Nitrobenzol)**

| Anilin [m%] | 4,4'-MDA [m%] | N-Formyl-MDA [m%] | 2,4'-MDA [m%] | N-Methyl-anilin [m%] | 3-Kerne* [m%] | 4-Kerne* | > 4-Kern* |
|---|---|---|---|---|---|---|---|
| 3,75 | 0,51 | 0,08 | 0,10 | 0,06 | 0,04 | 0,00 | 0,13 |

| | | | | | | | |
|---|---|---|---|---|---|---|---|
| höher kondensierte MDA-Spezies (z.B. 3-Kern= Reaktionsprodukt 3 Anilin + 2 Formaldehydeinheiten) | | | | | | | |

## Patentansprüche

1. Verfahren zur Herstellung von Di- und Polyaminen der Diphenylmethanreihe, bei dem man
a) Nitrobenzol und Methanol in Gegenwart eines Katalysators zu Anilin und Formaldehyd umsetzt, und anschließend
b) das in Schritt a) hergestellte Anilin und Formaldehyd in Gegenwart eines sauren Katalysators zu Di- und Polyaminen der Diphenylmethanreihe umsetzt.

2. Verfahren nach Anspruch 1, bei dem man in Schritt a) einen Katalysator einsetzt, der ein oder mehrere Metalle, ausgewählt aus der Gruppe Pd, Pt, Rh, Ir, Ru, Fe, Co, Ni, Cu, Al, Mg, Zr, Zn, V, Cr, Mo, W, Pb, Lanthanoide, als katalytisch wirksame Komponenten in elementarer oder gebundener Form enthalten.

3. Verfahren nach Anspruch 1 oder 2, bei dem Schritt a) in Gegenwart einer Base durchgerührt wird.

4. Verfahren nach einem der Ansprüche 1 bis 3, bei dem man aus dem in Schritt a) hergestellten Anilin und Formaldehyd das Anilin und/oder Formaldehyd teilweise entfernt.

5. Verfahren nach einem der Ansprüche 1 bis 4, bei dem man aus dem in Schritt a) hergestellten Anilin und Formaldehyd die Nebenprodukte Ameisensäure und/oder CO und/oder CO₂ und/oder Carbonate und/oder Ameisensäuremethylester und/oder N-Formylanilin und/oder N-Methylanilin teilweise oder vollständig abtrennt.

6. Verfahren nach einem der Ansprüche 1 bis 5, bei dem als saurer Katalysator in Schritt b) Salzsäure eingesetzt wird.

7. Verfahren zur Herstellung von Di- und Polyisocyanaten der Diphenylmethanreihe, bei dem man
a) Nitrobenzol und Methanol in Gegenwart eines Katalysators zu Anilin und Formaldehyd umsetzt, und anschließend
b) das in Schritt a) hergestellte Anilin und Formaldehyd in Gegenwart eines sauren Katalysators zu Di- und Polyaminen der Diphenylmethanreihe umsetzt, und anschließend
c) die in Schritt b) hergestellten Di- und Polyamine der Diphenylmethanreihe durch Phosgenierung zu den Di- und Polyisocyanaten der Diphenylmethanreihe umsetzt.

## Claims

1. Process for the preparation of diamines and polyamines of the diphenylmethane series, wherein
a) nitrobenzene and methanol are converted to aniline and formaldehyde in the presence of a catalyst, and then
b) the aniline and formaldehyde prepared in step a) are converted to diamines and polyamines of the diphenylmethane series in the presence of an acidic catalyst.

2. Process according to Claim 1 wherein the catalyst used in step a) contains one or more metals selected from the group comprising Pd, Pt, Rh, Ir, Ru, Fe, Co, Ni, Cu, A1, Mg, Zr, Zn, V, Cr, Mo, W, Pb and lanthanoids, as catalytically active components in elemental or bound form.

3. Process according to Claim 1 or 2 wherein step a) is carried out in the presence of a base.

4. Process according to one of Claims 1 to 3 wherein the aniline and/or formaldehyde are partially removed from the aniline and formaldehyde prepared in step a).

5. Process according to one of Claims 1 to 4 wherein the by-products formic acid and/or CO and/or CO₂ and/or carbonates and/or methyl formate and/or N-formylaniline and/or N-methylaniline are partially or completely separated from the aniline and formaldehyde prepared in step a).

6. Process according to one of Claims 1 to 5 wherein hydrochloric acid is used as the acidic catalyst in step b).

7. Process for the preparation of diisocyanates and polyisocyanates of the diphenylmethane series, wherein
a) nitrobenzene and methanol are converted to aniline and formaldehyde in the presence of a catalyst, then
b) the aniline and formaldehyde prepared in step a) are converted to diamines and polyamines of the diphenylmethane series in the presence of an acidic catalyst, and then
c) the diamines and polyamines of the diphenylmethane series prepared in step b) are converted to diisocyanates and polyisocyanates of the diphenylmethane series by phosgenation.

## Revendications

1. Procédé de préparation de di- et polyamines de la série du diphénylméthane, dans lequel
a) on transforme le nitrobenzène et le méthanol en aniline et en formaldéhyde en présence d'un catalyseur et
b) on transforme ensuite l'aniline et le formaldéhyde préparés dans l'étape a) en di- et polyamines de la série du diphénylméthane en présence d'un catalyseur acide.

2. Procédé selon la revendication 1, dans lequel on met en oeuvre dans l'étape a) un catalyseur qui contient comme composants exerçant une activité catalytique, sous forme élémentaire ou combinée, un ou plusieurs métaux sélectionnés parmi le groupe formé par le palladium, le platine, le rhodium, l'iridium, le ruthénium, le fer, le cobalt, le nickel, le cuivre, l'aluminium, le magnésium, le zirconium, le zinc, le vanadium, le chrome, le molybdène, le tungstène et le plomb, ainsi que les lanthanides.

3. Procédé selon la revendication 1 ou 2, dans lequel l'étape a) est réalisée en présence d'une base.

4. Procédé selon une des revendications 1 à 3, dans lequel on élimine partiellement l'aniline et/ou le formaldéhyde de l'aniline et du formaldéhyde préparés dans l'étape a).

5. Procédé selon une des revendications 1 à 4, dans lequel on sépare partiellement ou totalement de l'aniline et du formaldéhyde préparés dans l'étape a) les produits secondaires - acide formique et/ou CO et/ou CO₂ et/ou carbonates et/ou esters méthyliques de l'acide formique et/ou N-formylaniline et/ou N-méthylaniline.

6. Procédé selon une des revendications 1 à 5, dans lequel on met en oeuvre de l'acide chlorhydrique comme catalyseur acide dans l'étape b).

7. Procédé de préparation de di- et polyamines de la série du diphénylméthane, dans lequel
a) on transforme le nitrobenzène et le méthanol en aniline et en formaldéhyde en présence d'un catalyseur et
b) on transforme ensuite l'aniline et le formaldéhyde préparés dans l'étape a) en di- et polyamines de la série du diphénylméthane en présence d'un catalyseur acide et on transforme ensuite
c) les di- et polyamines de la série du diphénylméthane préparées dans l'étape b) par phosgénation en di- et polyisocyanates de la série du diphénylméthane.
